# EUROPEAN PATENT APPLICATION

(11) **EP 4 692 993 A1**
(43) Date of publication of application: **11.02.2026**
(21) Application number: 24802515.7
(22) Date of filing: 10.01.2024
(51) Int. Cl.: G06F 3/01

(54) **EYE MOVEMENT TRACKING METHOD, READABLE STORAGE MEDIUM, PROGRAM PRODUCT AND ELECTRONIC DEVICE**

(30) Priority: 08.05.2023 CN 202310511960
(71) Applicant: Huawei Technologies Co., Ltd., Shenzhen, Guangdong 518129 (CN)
(72) Inventor: GAO, Shengjie, Shenzhen, Guangdong 518129 (CN); CHAI, Xin, Shenzhen, Guangdong 518129 (CN)
(74) Representative: Goddar, Heinz J.
(86) International application number: PCT/CN2024/071604
(87) International publication number: WO 2024/230227

(57) **Abstract**

This application relates to the field of terminal technologies, and discloses an eye movement tracking method, a readable storage medium, a program product, and an electronic device. The method is applied to an electronic device including a plurality of light sources. After capturing a frame of eye image in an eye movement tracking process, the electronic device may dynamically adjust, based on at least one of an image parameter of the frame of eye image, an eye movement manner of a user determined based on the frame of eye image, and a projection parameter of a light source projection in the frame of eye image, a light emitting parameter of each light source when the electronic device captures a next frame of eye image or a plurality of frames of eye image. For example, the electronic device adjusts light source brightness, or turns on/turns off a part of light sources. In this way, power consumption of the light sources in the electronic device can be reduced and eye movement tracking precision can be improved in some scenarios.

## Description

This application claims priority to Chinese Patent Application No. 202310511960.3, filed with the China National Intellectual Property Administration on May 8, 2023 and entitled "EYE MOVEMENT TRACKING METHOD, READABLE STORAGE MEDIUM, PROGRAM PRODUCT, AND ELECTRONIC DEVICE", which is incorporated herein by reference in its entirety.

### TECHNICAL FIELD

This application relates to the field of terminal technologies, and in particular, to an eye movement tracking method, a readable storage medium, a program product, and an electronic device.

### BACKGROUND

An extended reality (extended range, XR) technology may include virtual reality (virtual reality, VR), augmented reality (augmented reality, AR), mixed reality (mixed reality, MR), and the like, and is widely used for display of various electronic devices, for example, a helmet mounted display (helmet mounted display, HMD) like VR glasses and a VR helmet. An electronic device that uses the XR technology is usually equipped with a light source that can emit light to a user's eye and a camera that is used to capture an eye image of a user, and an eye movement manner, a fixation point, and the like of the user are detected based on a position relationship between a projection of a light source in the eye image and a user's eyeball, to adjust a display parameter of the electronic device, so that a picture displayed by the electronic device matches the eye movement manner or the fixation point of the user.

However, because eye movement tracking needs to be continuously performed when the user uses the electronic device that uses the XR technology, the light source used to emit light to the user's eye is always in an on state during use of the electronic device by the user. As a result, power consumption is high, and a battery life of the electronic device is affected.

### SUMMARY

In view of this, this application provides an eye movement tracking method, a readable storage medium, a program product, and an electronic device. In an eye movement tracking process, the electronic device may dynamically adjust a light emitting parameter of each light source, to reduce power consumption of the light source and improve a battery life of the electronic device.

According to a first aspect, this application provides an eye movement tracking method, applied to an electronic device. The electronic device includes a plurality of light sources. The method includes: capturing a first eye image of a user, where the first eye image includes a projection of at least one of the plurality of light sources onto the user's eye; adjusting a light emitting parameter of at least a part of the plurality of light sources based on the first eye image; and capturing a second eye image, and performing eye movement tracking based on the second eye image.

In other words, during eye movement tracking, the electronic device may adjust, based on a first eye image captured at a moment (for example, a current frame of eye image in the following), a light emitting parameter of a light source of the electronic device when the electronic device captures a second eye image after the moment (for example, a next frame of eye image in the following). For example, the electronic device may reduce a quantity of light sources that emit light, or reduce light emitting brightness of the light sources that emit light. In this way, power consumed by the light source can be reduced, and a battery life of the electronic device can be improved.

In a possible implementation of the first aspect, the light emitting parameter includes at least one of the following parameters: light emitting brightness and on/off.

In a possible implementation of the first aspect, the adjusting the light emitting parameter of the at least a part of the plurality of light sources based on the first eye image includes: adjusting the light emitting parameter of the at least a part of the plurality of light sources in at least one of the following manners: adjusting light emitting brightness of the at least a part of the plurality of light sources based on an image parameter of the first eye image, or turn-on/turn-off of the at least a part of light sources, where the image parameter includes contrast; controlling turn-on or turn-off of the at least a part of the plurality of light sources based on a projection parameter of each projection, where the projection parameter includes at least one of a distribution, an area, brightness, or a shape; and controlling turn-on or turn-off of the at least a part of the plurality of light sources based on a movement status of the user's eye, where the movement status is obtained based on the first eye image.

In a possible implementation of the first aspect, the adjusting the light emitting brightness of the at least a part of the plurality of light sources based on the image parameter of the first eye image, or turn-on/turn-off of the at least a part of light sources includes: when the contrast of the first eye image is greater than first contrast, reducing light emitting brightness of the at least one light source, or turning off a part of light sources in the at least one light source; or when the contrast of the first eye image is less than second contrast, increasing light emitting brightness of the at least one light source, or increasing a quantity of light sources that are turned on.

In other words, if the contrast of the first eye image is greater than the first contrast, the electronic device may reduce light emitting brightness of the at least one light source, or turn off a part of the at least one light source. In this way, power consumed by the light source can be reduced, and a battery life of the electronic device can be improved. If the contrast of the first eye image is less than the second contrast, the electronic device may increase the light emitting brightness of the at least one light source, or increase the quantity of turned-on light sources. In this way, contrast of the second eye image can be improved, and eye movement tracking precision can be improved.

In a possible implementation of the first aspect, the controlling turn-on or turn-off of the at least a part of the plurality of light sources based on the projection parameter of each projection includes: determining an invalid projection in the first eye image, and turning off a light source corresponding to the invalid projection.

In other words, when the invalid projection exists in the first eye image, the electronic device may turn off the light source corresponding to the invalid projection, so that power consumption of the light source can be reduced, and the invalid projection does not affect eye movement tracking precision.

In a possible implementation of the first aspect, the determining the invalid projection in the first eye image includes: determining a first projection as the invalid projection when the first projection meets at least one of the following conditions: in the first eye image, a distance between the first projection and a center of the user's eye is greater than a first distance; in the first eye image, an area of a connected region formed by the first projection and a second projection corresponding to at least one second light source is greater than a first area, or a distance between the connected region and the center of the user's eye is greater than a second distance; an area of the first projection is less than a second area; brightness of the first projection is less than first brightness; and a similarity between a shape of the first projection and a first shape is less than a first similarity.

In a possible implementation of the first aspect, the adjusting the light emitting parameter of the at least a part of light sources in the plurality of light sources based on the movement status of the user's eye includes: adjusting a quantity of turned-on light sources in the plurality of light sources to a first quantity when an eye movement manner of the user is fixation; or adjusting a quantity of turned-on light sources in the plurality of light sources to a second quantity when an eye movement manner of the user is a saccade or a smooth pursuit, where the first quantity is less than the second quantity.

In other words, when the eye movement manner of the user is fixation, the electronic device may reduce the quantity of turned-on light sources, to reduce power consumption of the light sources and improve a battery life of the electronic device.

In a possible implementation of the first aspect, the method further includes: adjusting a quantity of turned-on light sources in the plurality of light sources to a third quantity when a distance between a first position of a pupil center of the user in the first eye image and a second position of the pupil center of the user in a third eye image is less than a third distance; or adjusting a quantity of turned-on light sources in the plurality of light sources to a fourth quantity when a distance between a first position and a second position is greater than or equal to a third distance, where the third quantity is less than the fourth quantity, and the third eye image is an eye image captured before the first eye image.

In other words, the electronic device may adjust the quantity of turned-on light sources based on a change of the pupil center of the user (where the change reflects the movement status of the user's eye), to reduce the quantity of turned-on light sources when the user's eye moves slowly, so as to reduce power consumption of the light sources and improve a battery life of the electronic device.

In a possible implementation of the first aspect, the second eye image includes a plurality of frames of eye image, and the method further includes: controlling the plurality of light sources to blink in a process of capturing the plurality of frames of eye image, and determining a model of the user's eye based on the second eye image.

In other words, when the second eye image includes the plurality of frames of eye image that are used to determine the model of the user's eye, the electronic device may control the plurality of light sources to blink in a process of capturing the plurality of frames of eye image, to capture eye images with different contrast. This helps improve precision of the determined model of the user's eye, and improve eye movement tracking precision.

In a possible implementation of the first aspect, a frequency at which the plurality of light sources blink is less than a capture frequency of the plurality of frames of eye image.

According to a second aspect, this application provides a readable storage medium. The readable storage medium includes an instruction. When the instruction is executed by an electronic device, the electronic device is enabled to implement any eye movement tracking method according to any one of the first aspect and the possible implementations of the first aspect.

According to a third aspect, this application provides an electronic device. The electronic device includes a plurality of light sources, a memory, configured to store instructions, and a processor, configured to execute the instructions, so that the electronic device controls the plurality of light sources to implement any eye movement tracking method according to any one of the first aspect and the possible implementations of the first aspect.

According to a fourth aspect, this application provides a program product. When the program product runs on an electronic device, the electronic device is enabled to implement any eye movement tracking method according to any one of the first aspect and the possible implementations of the first aspect.

It may be understood that, for beneficial effects of the second aspect to the fourth aspect, reference may be made to the descriptions of the first aspect. Details are not described herein again.

### BRIEF DESCRIPTION OF DRAWINGS

FIG. 1 is a diagram of an eye movement tracking scenario according to some embodiments of this application;
FIG. 2A is a diagram of an eye image according to some embodiments of this application;
FIG. 2B is a diagram of another eye image according to some embodiments of this application;
FIG. 2C is a diagram of still another eye image according to some embodiments of this application;
FIG. 3 is a diagram of a process of adjusting a light emitting parameter of a light source in an eye movement manner of a user according to some embodiments of this application;
FIG. 4A is a diagram of a current frame of eye image and a next frame of eye image according to some embodiments of this application;
FIG. 4B is a diagram of another current frame of eye image and next frame of eye image according to some embodiments of this application;
FIG. 4C is a diagram of still another current frame of eye image and next frame of eye image according to some embodiments of this application;
FIG. 4D is a diagram of yet another current frame of eye image and next frame of eye image according to some embodiments of this application;
FIG. 5 is a diagram of a structure of VR glasses 10 according to some embodiments of this application;
FIG. 6A is a diagram of distribution of light sources in a light source group 11L/light source group 11R according to some embodiments of this application;
FIG. 6B is a diagram of another distribution of light sources in the light source group 11L/light source group 11R according to some embodiments of this application;
FIG. 7 is a schematic flowchart of an eye movement tracking method according to some embodiments of this application;
FIG. 8A is a diagram of a light source switch status in a case shown in FIG. 2B according to some embodiments of this application;
FIG. 8B is a diagram of a light source switch status in a case shown in FIG. 2C according to some embodiments of this application;
FIG. 9 is a diagram of a process of adjusting a light emitting parameter of a light source based on a pupil position of a user according to some embodiments of this application;
FIG. 10A is a schematic flowchart of another eye movement tracking method according to some embodiments of this application;
FIG. 10B is a diagram of capturing a calibrated eye image according to some embodiments of this application;
FIG. 11A is a diagram of a process of an eye movement tracking method according to some embodiments of this application; and
FIG. 11B is a diagram of a control time sequence of an eye movement tracking method according to some embodiments of this application.

### DESCRIPTION OF EMBODIMENTS

Illustrative embodiments of this application include but are not limited to an eye movement tracking method, a readable storage medium, a program product, and an electronic device.

The following describes technical solutions in this application with reference to accompanying drawings.

It may be understood that eye movement tracking, also referred to as eyeball tracking, is to detect a movement status of a user's eyeball relative to the user's head or an electronic device and/or a fixation point of the user's eye, so that the electronic device may adjust a display parameter based on a tracking result, to make a picture displayed by the electronic device match the movement status or the fixation point of the user's eye.

It may be understood that the technical solutions of this application are applicable to any electronic device that performs eye movement tracking by capturing projections of a plurality of light sources in the user's eye, and the electronic device includes but is not limited to an HMD, a head-up display (head-up display, HUD) device, and the like, for example, a VR/AR/MR apparatus of a headphone, a helmet, goggles, or glasses. For ease of description, the following describes the technical solutions of this application by using an example in which the electronic device is VR glasses.

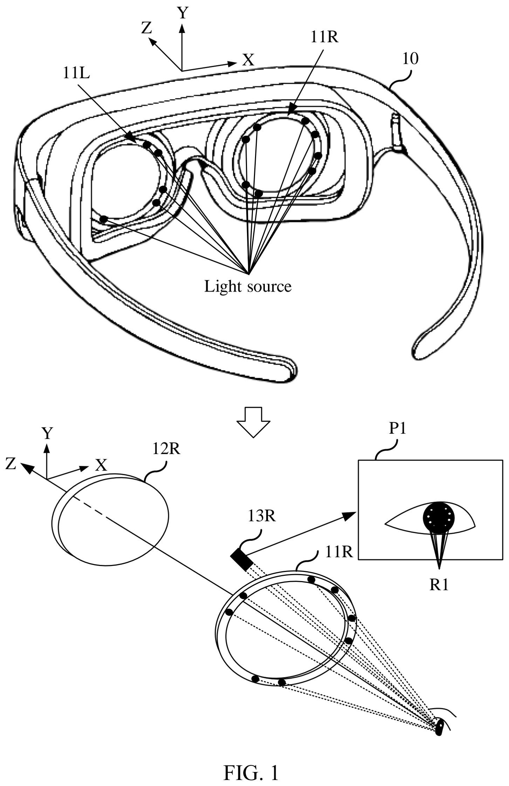

FIG. 1 is a diagram of an eye movement tracking scenario according to some embodiments of this application.

As shown in FIG. 1, VR glasses 10 include a light source group 11L and a light source group 11R. The light source group 11L includes a plurality of light sources, and is configured to emit light to a user's left eye when a user wears the VR glasses 10, to project a light source of the light source group 11L onto the user's left eye. The light source group 11R includes a plurality of light sources, and is configured to emit light to a user's right eye when the user wears the VR glasses 10, to project a light source of the light source group 11R onto the user's right eye. The VR glasses 10 further include a camera configured to capture an eye image of the user, for example, a camera 13L (not shown) configured to capture an eye image of the user's left eye and a camera 13R configured to capture an eye image of the user's right eye, and a display 12L (not shown) and a display 12R that are configured to display a display image.

It may be understood that an eye movement tracking apparatus (including at least the light source group 11L and the camera 13L) for the user's left eye and an eye movement tracking apparatus (including at least the light source group 11R and the camera 13R) for the user's left eye in the VR glasses 10 may have a symmetrical structure. The following uses the eye movement tracking apparatus for the user's right eye as an example for description.

Refer to FIG. 1. For ease of description, it is defined that when the VR glasses 10 are worn by the user, a horizontal direction is an X direction, a vertical direction is a Y direction, and a direction perpendicular to an XY plane is a Z direction. The display 12R, the camera 13R, and the light source group 11R are sequentially arranged along the Z direction.

When the user wears the VR glasses 10 to view an image on the display 12R, the light source in the light source group 11R emits light to the user's right eye, to project at least a part of light sources in the light source group 11R onto the user's right eye. After the user's eye receives the light sent by the light source group 11R, the received light may be reflected to the camera 13R, so that the camera 13R captures an eye image P1 of the user's right eye. The eye image P1 includes an eye image of the user and a projection R1 of the at least a part of light sources in the light source group 11R in the user's eye. After obtaining the eye image P1, the VR glasses 10 may determine a movement status of the user's eye relative to the user's head or the VR glasses 10 based on a position relationship between the projection R1 and the user's eye, that is, perform eye movement tracking on the user's eye.

It may be understood that an arrangement manner of the display 12R, the camera 13R, and the light source group 11R shown in FIG. 1 is merely an example. In some other implementations, the display 12R, the camera 13R, and the light source group 11R may be arranged in another manner. This is not limited herein.

However, in a process in which the user uses the VR glasses 10, the VR glasses 10 usually keep all light sources in the light source group 11L and the light source group 11R turned on. This increases power consumption of the light source group 11L and the light source group 11R, and reduces a battery life of the VR glasses 10.

It may be understood that in some scenarios, a change of an image parameter (for example, contrast) of the eye image within an allowable range of an eye movement tracking algorithm does not affect eye movement tracking precision. In addition, when other factors remain the same, higher brightness of a light source indicates higher contrast of an image captured by a camera and higher power consumption of the light source. Therefore, when contrast of a captured eye image is high, the VR glasses 10 may reduce brightness of the light source, to reduce power consumption of the light source group 11L and the light source group 11R.

It may be understood that in some scenarios, due to different shapes, sizes, and the like of eyes of different users, projections of a part of light sources in the users' eyes cannot improve eye movement tracking precision or reduce eye movement tracking precision (the projections are referred to as invalid projections below). Therefore, the VR glasses 10 may turn off light sources corresponding to the invalid projections, to reduce power consumption of the light source group 11L and the light source group 11R.

It may be understood that in some scenarios, corresponding to different movement statuses (for example, a running speed and an eye movement manner) of the user's eye, there are different quantities of reference objects (projections of light sources) required by the eye movement tracking algorithm to achieve same tracking precision. For example, a higher running speed of the user's eye indicates a larger quantity of required reference objects. Therefore, the VR glasses 10 may turn on different quantities of light sources based on the movement statuses of the user's eye, to reduce a quantity of turned-on light sources when a dynamic speed of the user's eye is low, and reduce power consumption of the light source group 11L and the light source group 11R.

In view of this, an embodiment of this application provides an eye movement tracking method. In a process in which the VR glasses 10 performs eye movement tracking, after capturing a frame of eye image (referred to as a current frame of eye image and used as a first eye image), the VR glasses 10 may dynamically adjust, based on at least one of an image parameter of the current frame of eye image, a movement status of a user determined based on the current frame of eye image, and a projection parameter (for example, a projection distribution, a projection area, projection brightness, or a projection shape) of a light source projection in the current frame of eye image, a light emitting parameter of at least a part of light sources in the light source group 11L and the light source group 11R when the VR glasses 10 capture a next frame of eye image or a plurality of next frames of eye image (used as a second eye image), for example, reduce light source brightness or turn off a part of light sources.

In this way, because higher light emitting brightness of a light source indicates higher consumed electric energy, in some scenarios, power consumption of the light source group 11L and the light source group 11R can be reduced. This improves a battery life of the VR glasses 10.

In some implementations, when contrast of the current frame of eye image is greater than first contrast, the VR glasses 10 may reduce brightness of at least a part of light sources in the light source group 11L and the light source group 11R, to reduce contrast of a next frame of captured eye image, so as to reduce electric energy consumption of the light source group 11L and the light source group 11R.

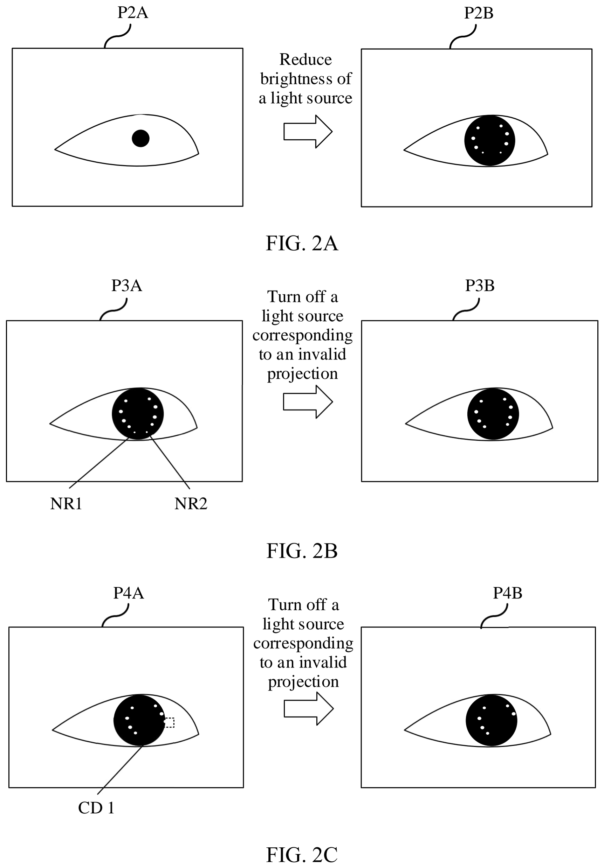

Refer to FIG. 2A. It is assumed that contrast of a current frame of eye image P2A captured by the camera 13R is greater than the first contrast. The VR glasses 10 may reduce brightness of at least a part of light sources that are in the light source group 11R and that are used when the camera 13R captures a next frame of eye image. As shown in FIG. 2A, contrast of the next frame of eye image P2B captured by the camera 13R is lower than that of the current frame of eye image P2A. In this way, electric energy consumption of the light source group 11R can be reduced, and a battery life of the VR glasses 10 can be improved.

It may be understood that when contrast of an eye image is excessively high, accuracy of a light source projection determined by the VR glasses 10 is reduced, and eye movement tracking precision is affected. Therefore, reducing light source brightness when the contrast of the eye image is greater than the first contrast helps improve eye movement tracking precision.

It may be understood that in some embodiments, when the contrast of the eye image is excessively low, accuracy of a light source projection determined by the VR glasses 10 is also reduced, and eye movement tracking precision is affected. Therefore, when the contrast of the eye image is less than second contrast, the VR glasses 10 may also increase light source brightness or turn on more light sources, to improve eye movement tracking precision.

In some implementations, if an invalid projection exists in the current frame of eye image, the VR glasses 10 may turn off a light source corresponding to the invalid projection, to improve a battery life of the VR glasses 10. In other words, the VR glasses 10 may first determine the invalid projection in the current frame of eye image, and then turn off the light source corresponding to the invalid projection when capturing the next frame of eye image or the plurality of frames of eye image.

It may be understood that, in some embodiments, the VR glasses 10 may determine the projection as the invalid projection if a projection in the eye image meets at least one of the following turn-off conditions.

A distance between the projection in the eye image and a center of the user's eye is greater than a first distance.

An area of a connected region formed by the projection in the eye image and at least one other projection is greater than a first area, or a distance between a center of the connected region and the center of the user's eye is greater than a second distance. It may be understood that the connected region is a set of a plurality of pixels with same or similar values in an image.

An area of the projection in the eye image is less than a second area.

Brightness of the projection in the eye image is less than second brightness.

A similarity between a shape in the eye image and a first shape is less than a first similarity. It may be understood that the first shape is a shape of a valid projection, for example, a circle, an ellipse, or a polygon, and the first shape may be obtained through experience or an experiment. A specific shape of the first shape is not limited in embodiments of this application.

For example, refer to FIG. 2B. A distance between the center of the human eye and each of a projection NR1 and a projection NR2 in a current frame of eye image P3A captured by the camera 13R is greater than the first distance, and the VR glasses 10 may turn off light sources corresponding to the projection NR1 and the projection NR2. As shown in FIG. 2B, compared with the current frame of eye image P3A, the eye image P3B captured by the camera 13R after the current frame of eye image P3A is captured does not include an invalid projection. In other words, when the camera 13R captures the eye image P3B, light sources corresponding to the projection NR1 and the projection NR2 are turned off. This reduces power consumption of the light source group 11R.

For another example, refer to FIG. 2C. A plurality of projections form a connected region CD 1 in the current frame of eye image P4A captured by the camera 13R. If an area of the connected region CD 1 is greater than the first area, the VR glasses 10 may determine the plurality of projections as invalid projections, and turn off a light source corresponding to the connected region CD 1. As shown in FIG. 2C, compared with the current frame of eye image P4A, the eye image P4B captured by the camera 13R after the current frame of eye image P4A is captured does not include an invalid projection. In other words, when the camera 13R captures the eye image P4B, the light source corresponding to the projection in the connected region CD 1 are turned off. This reduces power consumption of the light source group 11R.

It may be understood that, in some other embodiments, the VR glasses 10 may alternatively determine the invalid projection in another manner, for example, determine the invalid projection based on judgment logic of an eye movement tracking algorithm used by the VR glasses 10. The manner of determining the invalid projection is not limited herein.

It may be understood that, in some implementations, a movement status of the user's eye may be represented in an eye movement manner. The eye movement manner may include fixation (fixation), saccade (saccade), and smooth pursuit (smooth pursuit). In this way, the VR glasses 10 may determine an eye movement manner of the user based on the current frame of eye image, and adjust brightness of at least a part of light sources in the light source group 11L and the light source group 11R based on the determined eye movement manner. For example, it is assumed that the light source group 11L and the light source group 11R each include N (N>1) light sources. When the eye movement manner of the user is fixation, M light sources in each light source group may be turned on. When the eye movement manner of the user is a saccade or a smooth pursuit, P light sources in each light source group may be turned on, where M<P≤N.

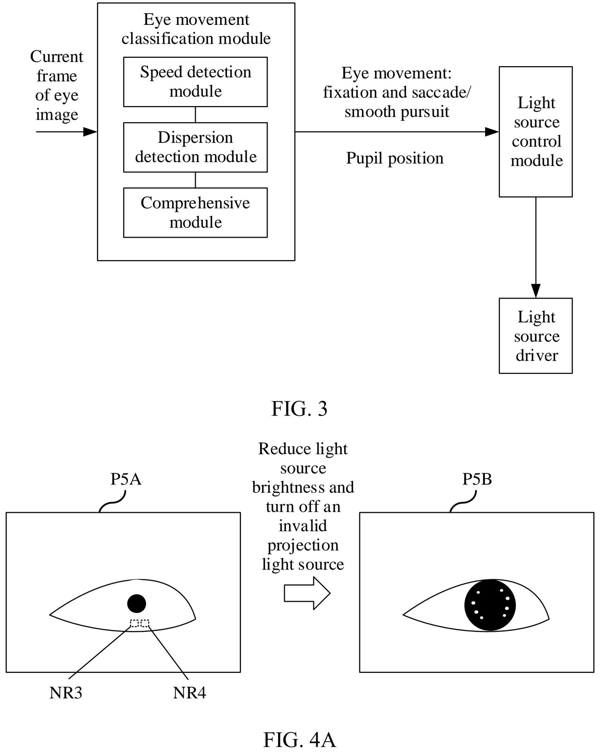

In some implementations, the VR glasses 10 may determine the eye movement manner by using an eye movement classification module. Refer to FIG. 3. The VR glasses 10 may input the current frame of eye image into the eye movement classification module to obtain the eye movement manner and/or a pupil position of the user, and send the eye movement manner and/or the pupil position of the user to a light source control module. The light source control module may determine, based on the eye movement manner and/or the pupil position of the user, a quantity of turned-on light sources used when the next frame of eye image is captured, and control, by using a light source driver, a switch status of each light source used when the next frame of eye image is captured.

For example, when the eye movement manner of the user is fixation, M light sources in each light source group may be turned on. When the eye movement manner of the user is a saccade or a smooth pursuit, P light sources in each light source group may be turned on.

It may be understood that the movement status of the user's eye may alternatively be represented by a change of a pupil of the user. Therefore, in some embodiments, the VR glasses 10 may adjust, based on a distance between a position of the pupil of the user in the current frame of eye image and a position of the pupil of the user in a previous frame of eye image, a light emitting parameter of each light source used when the next frame of eye image is captured. For example, when the distance is less than a third distance, M light sources in each light source group may be turned on; or when the distance is greater than or equal to the third distance, P light sources in each light source group may be turned on.

It may be understood that the eye movement classification module may include a speed detection module, a dispersion detection module, and a comprehensive module. The speed detection module is configured to determine a movement speed of the user's eye. The dispersion detection module is configured to determine dispersion of a projection in the current frame of eye image. The comprehensive module is configured to determine the eye movement manner or the pupil position of the user's eye based on the movement speed of the user's eye and dispersion of the projection.

In some implementations, when determining a human eye model, the VR glasses 10 may further control the light sources in the light source group 11L and the light source group 11R to blink, to improve validity of the light source projection in the captured eye image, and improve accuracy of the determined human eye model.

It may be understood that, in some other embodiments, the VR glasses 10 may alternatively adjust the light emitting parameters of the light sources in the light source group 11L and the light source group 11R in combination with the foregoing plurality of manners.

For example, the VR glasses 10 may adjust brightness of the light sources in the light source group 11L and the light source group 11R based on the image parameter of the current frame of eye image, and turn off, based on the invalid projection in the current frame of eye image, the light source corresponding to the invalid projection. Refer to FIG. 4A. A distance between the center of the human eye and each of a projection NR3 and a projection NR4 in a current frame of eye image P5A captured by the camera 13R is greater than the first distance, and contrast of the current frame of eye image P5A is greater than first contrast. When the camera 13R captures a next frame of eye image P5B, light sources corresponding to the projection NR3 and the projection NR4 may be turned off and brightness of the turned-on light sources may be reduced.

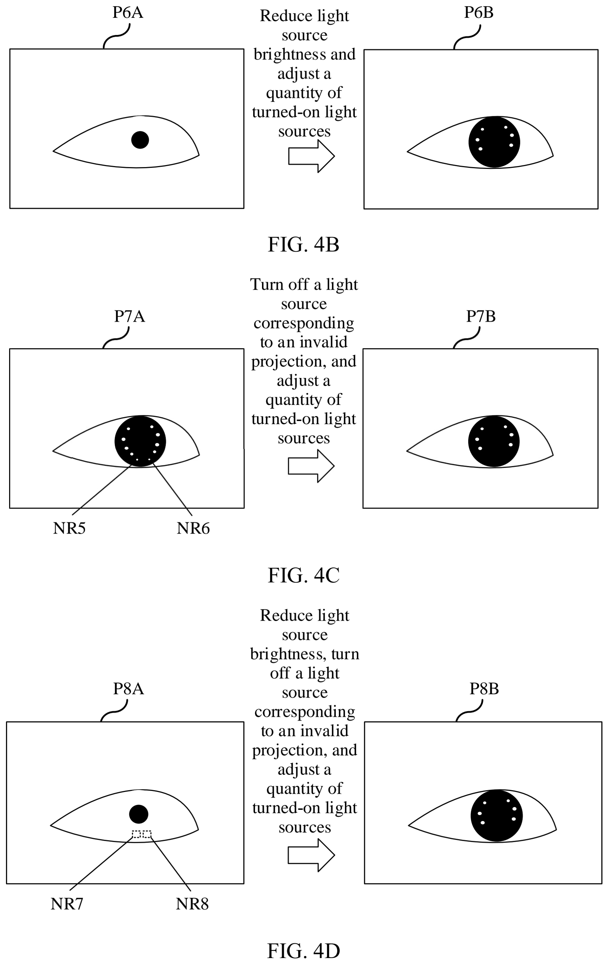

For another example, the VR glasses 10 may adjust the brightness of the light sources in the light source group 11L and the light source group 11R based on the image parameter of the current frame of eye image, and turn on or off a corresponding quantity of light sources based on a movement status of the user's eye. Refer to FIG. 4B. Contrast of a current frame of eye image P6A captured by the camera 13R is greater than the first contrast, and it is determined that the eye movement manner of the user is fixation. When the camera 13R captures a next frame of eye image P6B, a quantity of turned-on light sources may be reduced to a quantity (for example, six) corresponding to the fixation, and brightness of the turned-on light sources may be reduced.

For still another example, the VR glasses 10 may turn off, based on the invalid projection in the current frame of eye image, the light source corresponding to the invalid projection, and turn on or off a corresponding quantity of light sources based on the movement status of the user's eye. Refer to FIG. 4C. A distance between the center of the human eye and each of a projection NR5 and a projection NR6 in a current frame of eye image P7A captured by the camera 13R is greater than a first distance, and it is determined that the eye movement manner of the user is fixation. When the camera 13R captures a next frame of eye image P7B, light sources corresponding to the projection NR5 and the projection NR6 may be turned off, a quantity of turned-on light sources may be reduced to a quantity (for example, six) corresponding to the fixation, and brightness of the turned-on light sources may be reduced.

For another example, the VR glasses 10 may adjust brightness of the light sources in the light source group 11L and the light source group 11R based on the image parameter of the current frame of eye image, turn off, based on the invalid projection in the current frame of eye image, the light source corresponding to the invalid projection, and turn on or off a corresponding quantity of light sources based on the movement status of the user's eyes. Refer to FIG. 4D. A distance between the center of the human eye and each of a projection NR7 and a projection NR8 in a current frame of eye image P8A captured by the camera 13R is greater than the first distance, contrast of the current frame of eye image P8A is greater than first contrast, and it is determined that the eye movement manner of the user is the fixation. When the camera 13R captures a next frame of eye image P8B, light sources corresponding to the projection NR7 and the projection NR8 may be turned off, a quantity of turned-on light sources may be reduced to a quantity (for example, six) corresponding to the fixation, and brightness of the turned-on light sources may be reduced.

It may be understood that, in some other embodiments, the VR glasses 10 may alternatively dynamically adjust, based on the current frame of eye image in another manner, a light emitting parameter of a part of light sources in the light source group 11L and the light source group 11R when a next frame of eye image or a plurality of frames of eye image are captured. This is not limited herein.

The following describes the technical solutions of this application with reference to a structure of the VR glasses 10.

The structure of the VR glasses 10 is first described.

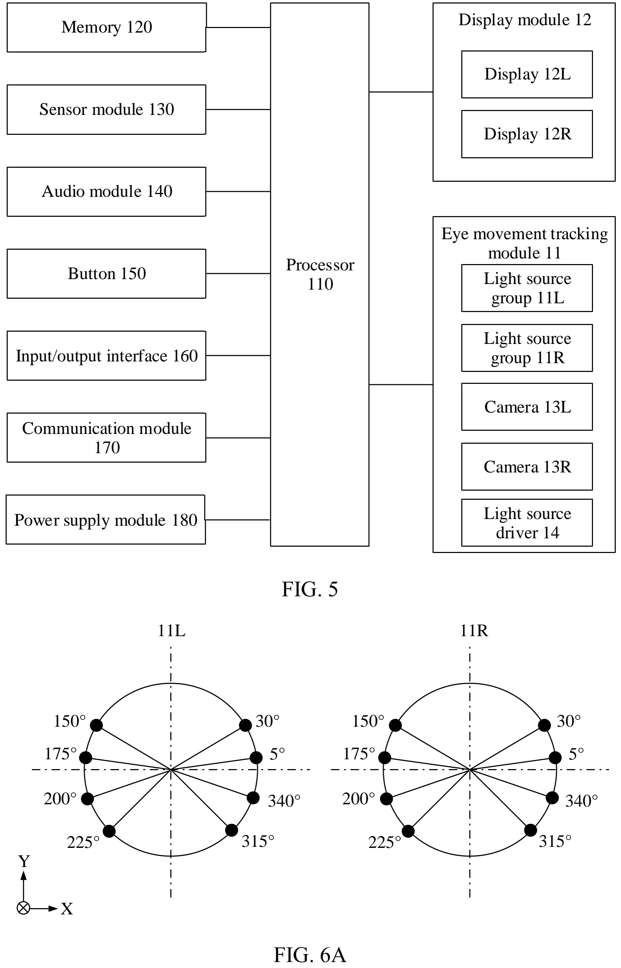

FIG. 5 is a block diagram of a structure of VR glasses 10 according to some embodiments of this application.

As shown in FIG. 5, the VR glasses 10 may include a processor 110, a memory 120, a sensor module 130, an audio module 140, a button 150, an input/output interface 160, a communication module 170, a power supply module 180, an eye movement tracking module 11, a display module 12, and the like.

The processor 110 is usually configured to control overall operations of the VR glasses 10, and may include one or more processing units. For example, the processor 110 may include an application processor (application processor, AP), a modem processor, a graphics processing unit (graphics processing unit, GPU), an image signal processor (image signal processor, ISP), a video processing unit (video processing unit, VPU) controller, a memory, a video codec, a digital signal processor (digital signal processor, DSP), a baseband processor, a neural-network processing unit (neural-network processing unit, NPU), and/or the like. Different processing units may be independent components, or may be integrated into one or more processors.

A memory may be further disposed in the processor 110, and is configured to store instructions and data. In some embodiments, the memory in the processor 110 is a cache memory. The memory may store instructions or data that has been used or cyclically used by the processor 110. If the processor 110 needs to use the instructions or the data again, the processor may directly invoke the instructions or the data from the memory. This avoids repeated access, reduces waiting time of the processor 110, and improves system efficiency.

In some embodiments, the processor 110 may be configured to run an instruction for adjusting, based on a current frame of eye image captured by the camera 13L and the camera 13R, light emitting parameters of the light sources that are in the light source group 11L and the light source group 11R and that are used when the camera 13L and the camera 13R capture a next frame of eye image.

In some embodiments, the processor 10 may be further configured to run instructions of the foregoing eye movement classification module and the foregoing light source control module, to determine an eye movement manner and a pupil position of a user based on the current frame of eye image, and adjust, based on the determined eye movement manner or pupil position, the light emitting parameters of the light sources that are in the light source group 11L and the light source group 11R and that are used when the next frame of eye image is captured.

In some embodiments, the processor 110 may include one or more interfaces. The interface may include an inter-integrated circuit (inter-integrated circuit, I2C) interface, a universal asynchronous receiver/transmitter (universal asynchronous receiver/transmitter, UART) interface, a mobile industry processor interface (mobile industry processor interface, MIPI), a general purpose input/output (general purpose input/output, GPIO) interface and/or a universal serial bus (universal serial bus, USB) interface, a serial peripheral interface (serial peripheral interface, SPI) interface, and the like.

For example, in some embodiments, the processor 110 may be connected to a light source driver 14 by using the I2C interface, the GPIO interface, or the like, and send, by using the I2C interface or the GPIO interface, the instruction for adjusting the light emitting parameters of the light sources in the light source group 11L and the light source group 11R to the light source driver.

The memory 120 may be configured to store computer-executable program code. The executable program code includes instructions. The processor 110 runs the instructions stored in the memory 120, to execute various function applications of the VR glasses 10 and process data. The memory 120 may include a program storage area and a data storage area. The program storage area may store an operating system, an application required by at least one function (for example, an image display function or a video playing function), and the like. The data storage area may store data created in a process of using the VR glasses 10 (for example, an eye image captured in a process of performing eye movement tracking by the VR glasses 10), and the like. In addition, the memory 120 may include a high-speed random access memory, and may further include a non-volatile memory, for example, at least one magnetic disk storage device, a flash memory device, or a universal flash storage (universal flash storage, UFS).

The sensor module 130 may include a sensor configured to detect a working status and a use status of the VR glasses 10, including but not limited to an optical proximity sensor/a contact sensor configured to detect whether the user wears the VR glasses 10, an inertial sensor configured to detect a head movement status of the user, and the like.

The audio module 140 may include a speaker, a microphone, and the like, and is configured to implement an audio function.

There may be one or more buttons 150. The button 150 may be in a form of a button, a switch, a dial, and a touch or near-touch sensing device (for example, a touch sensor). VR glasses 10 can trigger a corresponding function based on a user's operation on one or more of the buttons 150, for example, adjusting a volume, or controlling the VR glasses 10 to enter a calibration mode.

The input/output interface 160 may connect another apparatus to the VR glasses 10 by using a proper component. The component may include, for example, an audio/video jack, a data connector, and the like. For example, in some embodiments, the eye movement tracking module 11 may not be a part of the VR glasses, but is connected to the VR glasses 10 by using the input/output interface 160 as an external device.

The communication module 170 may include a wireless communication module. The wireless communication function may be implemented by using an antenna (not shown), a modem processor (not shown), a baseband processor (not shown), and the like. The antenna is configured to transmit and receive electromagnetic wave signals. The VR glasses 10 may include a plurality of antennas, and each antenna may be configured to cover one or more communication frequency bands. Different antennas may be further multiplexed, to improve antenna utilization. For example, the antenna 1 may be multiplexed as a diversity antenna of a wireless local area network. In some other embodiments, the antenna may be used in combination with a tuning switch.

The wireless communication module may provide a solution for wireless communication that is applied to the VR glasses 10 and that includes a wireless local area network (wireless local area network, WLAN) (such as a wireless fidelity (wireless fidelity, Wi-Fi) network), Bluetooth (Bluetooth, BT), a global navigation satellite system (global navigation satellite system, GNSS), frequency modulation (frequency modulation, FM), near field communication (near field communication, NFC), an infrared (infrared, IR) technology, and the like. The wireless communication module may be one or more components that integrate at least one communication processing module. The wireless communication module receives an electromagnetic wave through an antenna, performs frequency modulation and filtering on an electromagnetic wave signal, and sends a processed signal to the processor 110. The wireless communication module may further receive a to-be-sent signal from the processor 110, perform frequency modulation and amplification on the to-be-sent signal, and convert the signal into an electromagnetic wave for radiation through the antenna.

The power supply module 180 may include a battery, configured to supply power to each module of the VR glasses 10.

The eye movement tracking module 11 may include a light source group 11L, a light source group 11R, a camera 13L, a camera 13R, a light source driver 14, and the like.

The light source group 11L is configured to emit light to a user's left eye, so that the camera 13L may capture an eye image of the user's left eye and a projection of a light source in the light source group 11L in the user's eye. The light source group 11R is configured to emit light to a user's right eye, so that the camera 13R can capture an eye image of the user's right eye and a projection of a light source in the light source group 11R in the user's eye.

It may be understood that the light source group 11L/the light source group 11R may include a plurality of light sources. For example, a quantity of light sources in each light source group may be 6, 7, 8, 10, or the like, or may be another quantity. This is not limited herein.

It may be understood that a light emitting wavelength of the light source may be an infrared band, a visible light band, or another band. This is not limited herein.

It can be understood that the light source in the light source group 11L or the light source group 11R may be a light-emitting diode (light-emitting diode, LED), an organic light-emitting diode (organic light-emitting diode, OLED), an active-matrix organic light-emitting diode (active-matrix organic light-emitting diode, AMOLED), a flexible light-emitting diode (flexible light-emitting diode, FLED), a mini-LED, a micro-LED, a micro-OLED, a quantum dot light-emitting diode (quantum dot light-emitting diodes, QLED), or the like. In some other embodiments, the light source in the light source group 11L and the light source in the light source group 11R may alternatively be light sources in another form. This is not limited herein.

It may be understood that the light source in the light source group 11L or the light source group 11R may be distributed on a circumference, an ellipse, a polygon, or a side of another irregular shape. This is not limited herein.

For example, in some embodiments, refer to FIG. 6A. The light source in the light source group 11L/the light source in the light source group 11R may include eight light sources, and the eight light sources are arranged on a same circumference. For example, the eight light sources in each light source group may be respectively arranged at positions of 5°, 30°, 150°, 175°, 200°, 225°, 315° and 340° that are on a circumference and whose positive half axis of the X axis is 0°.

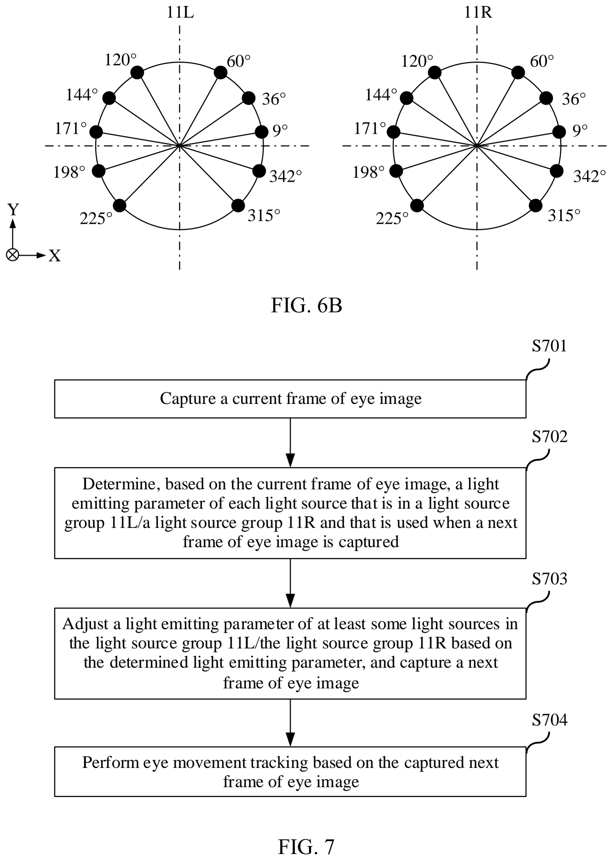

For another example, in some embodiments, refer to FIG. 6B. The light source in the light source group 11L/the light source in the light source group 11R may include 10 light sources, and the 10 light sources are arranged on a same circumference. For example, 10 light sources in each light source group may be respectively arranged at positions of 9°, 36°, 60°, 120°, 144°, 171°, 198°, 225°, 315°, and 342° that are on the circumference and whose positive half axis of the X axis is 0°.

The camera 13L/the camera 13R may be a camera that matches a light emitting wavelength of the light source in the light source group 11L/the light source in the light source group 11R, and is configured to capture the eye image of the user. For example, when the light source is a visible light source, the camera 13L/the camera 13R may be a camera in a visible light band; or when the light source is an infrared light source, the camera 13L/the camera 13R may be a visible infrared camera.

It may be understood that the camera 13L/the camera 13R may be disposed at any position that can capture the eye image of the user.

It may be understood that, in some embodiments, the VR glasses 10 may include only one camera that can simultaneously capture eye images of the user's left eye and right eye, or may capture eye images of the user by using more (for example, more than two) cameras. This is not limited herein.

The light source driver 14 is configured to adjust a light emitting parameter of each light source based on an instruction or a signal of the processor 110. For example, the light source driver 14 adjusts, based on the instruction or the signal of the processor 110, brightness, blink, and on/off of each light source by adjusting a voltage or a current applied to each light source.

It may be understood that the structure of the VR glasses 10 shown in FIG. 5 is merely an example. In some other embodiments, the VR glasses 10 may include more or fewer modules, or some modules may be combined or split. This is not limited herein.

The following describes the technical solutions in this application with reference to the structure of the VR glasses 10 shown in FIG. 5.

FIG. 7 is a schematic flowchart of an eye movement tracking method according to some embodiments of this application. The method may be performed by the VR glasses 10, for example, the processor 110 or another computing unit in the VR glasses. As shown in FIG. 7, the method may include the following steps.

S701: Capture a current frame of eye image.

The VR glasses 10 capture the current frame of eye image in an eye movement tracking process.

It may be understood that, in some embodiments, when the VR glasses 10 capture the current frame of eye image, the light emitting parameter of the light source in the light source group 11L/the light source group 11R may be determined based on a previous frame of eye image.

It may be understood that, in some embodiments, if the current frame of eye image is a first frame of eye image captured in a use process of the VR glasses 10, when the VR glasses 10 capture the current frame of eye image, the light emitting parameter of the light source in the light source group 11L/light source group 11R may be a preset light emitting parameter of the VR glasses 10. For example, all light sources emit light at same preset brightness.

It may be understood that, in some embodiments, in a use process of the VR glasses 10, the camera 13L and the camera 13R may capture eye images of the user at a same sampling frequency, for example, capture the eye images at a sampling frequency of 30 Hz to 160 Hz. For example, if the sampling frequencies of the camera 13L and the camera 13R are 45 Hz, the camera 13L and the camera 13R capture a frame of eye image every 1000 ms/45=22.2 ms. The current frame of eye image may be any frame of eye image captured by the camera 13L or the camera 13R.

It may be understood that, in some embodiments, after capturing the current frame of eye image, the VR glasses 10 may perform eye movement tracking based on the current frame of eye image, to determine a movement status of the user's eye or a fixation point of the user.

S702: Determine, based on the current frame of eye image, a light emitting parameter of each light source that is in the light source group 11L/light source group 11R and that is used when a next frame of eye image is captured.

After capturing the current frame of eye image, the VR glasses 10 may determine, based on at least one of an image parameter of the current frame of eye image, a movement status of the user's eye that is determined based on the current frame of eye image, and a projection parameter (for example, a projection distribution, a projection area, projection brightness, or a projection shape) of a light source projection in the current frame of eye image, the light emitting parameter of each light source that is in the light source group 11L/light source group 11R and that is used when the next frame of eye image is captured.

In some implementations, after capturing the current frame of eye image, the VR glasses 10 may first determine the image parameter of the current frame of eye image, for example, contrast. Then, the VR glasses 10 may compare the determined image parameter with a preset image parameter, and determine, based on a comparison result, the light emitting parameter of each light source that is in the light source group 11L/light source group 11R and that is used when the next frame of eye image is captured.

For example, when the contrast of the current frame of eye image is greater than first contrast, brightness of currently turned-on light sources in the light source group 11L and the light source group 11R is reduced, or some of currently turned-on light sources are turned off, to reduce contrast of the next frame of captured eye image, so as to reduce electric energy consumption of the light source group.

For another example, when the contrast of the current frame of eye image is less than second contrast, brightness of currently turned-on light sources in the light source group 11L and the light source group 11R is increased, or a quantity of turned-on light sources is increased, to increase contrast of the next frame of captured eye image, so as to improve eye movement tracking precision.

In some implementations, after capturing the current frame of eye image, the VR glasses 10 may first determine whether there is an invalid projection in the current frame of eye image; and if it is determined that there is the invalid projection in the current frame of eye image, turn off a light source corresponding to the invalid projection. In this way, power consumption of the VR glasses 10 can be reduced and eye movement tracking precision can be improved.

It may be understood that, in some embodiments, the VR glasses 10 may determine the projection as the invalid projection if a projection in the eye image meets at least one of the following turn-off conditions.

A distance between the projection in the eye image and a center of the user's eye is greater than a first distance.

An area of a connected region formed by the projection in the eye image and at least one other projection is greater than a first area, or a distance between a center of the connected region and the center of the human eye is greater than a second distance. It may be understood that the connected region is a set of a plurality of pixels with same or similar values in an image.

An area of the projection in the eye image is less than a second area.

Brightness of the projection in the eye image is less than second brightness.

A similarity between a shape in the eye image and a first shape is less than a first similarity. It may be understood that the first shape is a shape of a valid projection, for example, a circle, an ellipse, or a polygon, and the first shape may be obtained through experience or an experiment. A specific shape of the first shape is not limited in embodiments of this application.

For example, the light source in the light source group 11R is disposed in a manner shown in FIG. 6B.

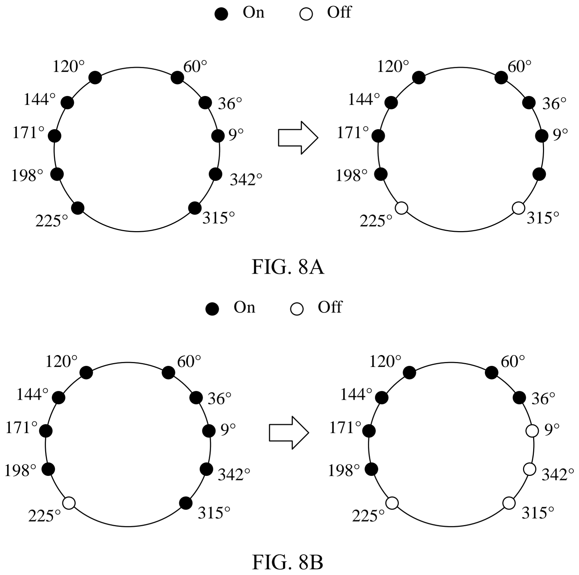

For the case shown in FIG. 2B, refer to FIG. 8A. All light sources in the light source group 11R are turned on when the current frame of eye image P3A is captured. Based on that the invalid projections NR1 and NR2 exist in the current frame of eye image P3A, and the invalid projections NR1 and NR2 respectively correspond to a light source at a position of 225° and a light source at a position of 315°, the VR glasses 10 may determine that the light source at the position of 225° and the light source at the position of 315° are turned off when the next frame of eye image P3B is captured.

For the case shown in FIG. 2C, refer to FIG. 8B. All light sources in the light source group 11R except the light source at the position of 225° are turned on when the current frame of eye image P4A is captured. Based on that the connected region CD 1 exists in the current frame of eye image P4A, the area of the connected region CD 1 is greater than the first area, and the projections in the connected region CD 1 respectively correspond to a light source at a position of 9°, a light source at a position of 315°, and a light source at a position of 342°, the VR glasses 10 may determine that the light source at the position of 9°, the light source at the position of 225°, the light source at the position of 315°, and the light source at the position of 342° are all turned off when the next frame of eye image P4B is captured.

In some implementations, after capturing the current frame of eye image, the VR glasses 10 may first determine an eye movement manner of the user's eye, and adjust, based on the eye movement manner of the user, a quantity of turned-on light sources in the light source group 11L/light source group 11R to a corresponding quantity. For example, it is assumed that the light source group 11L and the light source group 11R each include N (N>1) light sources. When the eye movement manner of the user is fixation, M light sources in each light source group may be turned on. When the eye movement manner of the user is a saccade or a smooth pursuit, P light sources in each light source group may be turned on, where M<P≤N.

In some implementations, after capturing the current frame of eye image, the VR glasses 10 may determine, based on a distance between a pupil position of the user in the current frame of eye image and a pupil position of the user in a previous frame of eye image, a quantity of turned-on light sources in the light source group 11L/light source group 11R.

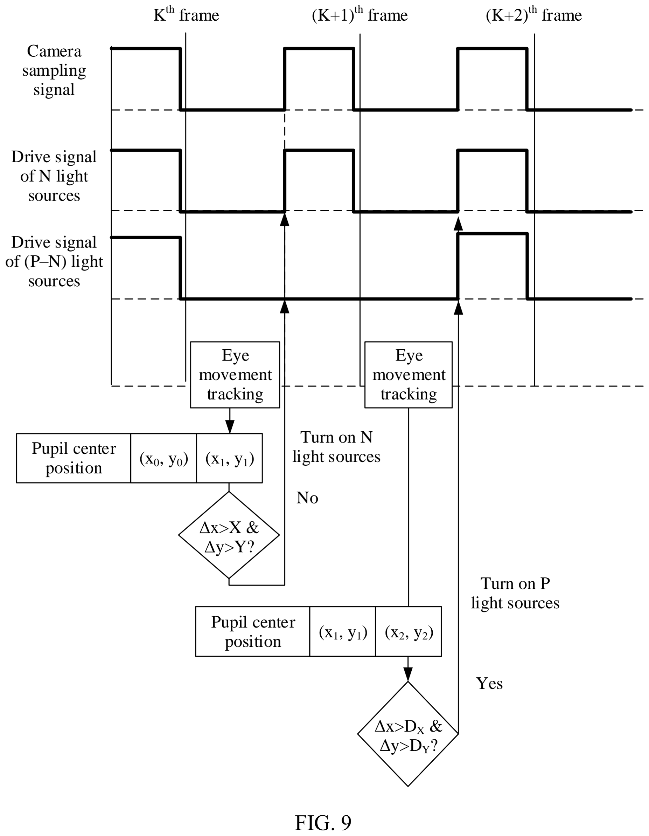

For example, refer to FIG. 9. When the current frame of eye image is the K^{th} frame of eye image captured by the camera 13R, the VR glasses 10 may determine that a pupil center position of the user's right eye in a K^{th} frame of eye image is (x₁, y₁). Then, the pupil center position (x₁, y₁) is compared with a pupil center position (x₀, y₀) of the user's right eye in a (K-1)^{th} frame of eye image, to determine a change amount Δx=|x₁-x₀| in an X direction and a change amount Δy=|y₁-y₀| in a Y direction of the pupil center position. Then, Δx and Δy are respectively compared with preset change amounts D_{X} and D_{Y}. When Δx≤D_{X} or Δy<D_{Y}, it is determined that N light sources are turned on when the (K+1)^{th} frame of eye image is captured.

Still refer to FIG. 9. When the current frame of eye image is the (K+1)^{th} frame of eye image captured by the camera 13R, the VR glasses 10 may determine that the pupil center position of the user's right eye in the (K+1)^{th} frame of eye image is (x₂, y₂). Then, the pupil center position (x₁, y₁) is compared with a pupil center position (x₁, y₁) of the user's right eye in the K^{th} frame of eye image, to determine a change amount Δx=|x₂-x₁| in the X direction and a change amount Δy=|y₂-y₁| in the Y direction of the pupil center position. Then, Δx and Δy are respectively compared with preset change amounts D_{X} and D_{Y}. When Δx>D_{X} and Δy>D_{Y}, it is determined that P light sources are turned on when a (K+2) ^{th} frame of eye image is captured.

It may be understood that, in some other embodiments, the VR glasses 10 may alternatively determine, when Δx<Dx and Δy≤D_{Y}, that N light sources are turned on when the (K+1)^{th} frame of eye image is captured; and determine, when Δx>Dx or Δy>D_{Y}, that P light sources are turned on when the (K+2)^{th} frame of eye image is captured. This is not limited herein.

It may be understood that, in some other embodiments, the VR glasses 10 may alternatively determine, in combination with a plurality of adjustment manners in the foregoing adjustment manners, a light emitting parameter of each light source that is in the light source group 11L/light source group 11R and that is used when a next frame of eye image is captured, or may determine, in another manner based on the current frame of eye image, the light emitting parameter of each light source that is in the light source group 11L/light source group 11R and that is used when the next frame of eye image is captured.

S703: Adjust a light emitting parameter of at least a part of light sources in the light source group 11L/light source group 11R based on the determined light emitting parameter, and capture the next frame of eye image.

After determining the light emitting parameter of each light source that is in the light source group 11L/light source group 11R and that is used when the next frame of eye image is captured, the VR glasses 10 adjust the light emitting parameter of the light source in the light source group 11L/light source group 11R to the determined light emitting parameter when capturing the next frame of eye image, and capture the next frame of eye image.

For example, when determining to adjust brightness of a part of light sources, the VR glasses 10 may input a corresponding digital or analog signal of the brightness to the light source driver 14, so that the light source driver 14 may output a corresponding current or voltage to each light source based on the received signal.

For another example, when determining to turn on or off a part of light sources, the VR glasses may input a corresponding turn-on or turn-off digital or analog level or turn-on or turn-off instruction to the light source driver 14, so that the light source driver 14 may turn on or off the corresponding light sources based on the received level or instruction. For example, refer to FIG. 9. When the (K+1)^{th} frame of eye image is captured, the processor 110 may output, to the light source driver 14, a high level corresponding to turning on N light sources and a high level corresponding to turning off P-N light sources.

For example, in the case shown in FIG. 2A, the VR glasses 10 may reduce brightness of at least a part of light sources that are in the light source group 11R and that are used when the camera 13R captures the next frame of eye image, and capture the next frame of eye image P2B.

For example, in the case shown in FIG. 2B, the VR glasses 10 may turn off light sources corresponding to the projection NR1 and the projection NR2, and capture the next frame of eye image P3B.

For example, in the case corresponding to FIG. 2C, the VR glasses 10 may turn off light sources corresponding to projections in the connected region CD 1, and capture the next frame of eye image P4B.

It may be understood that, in some embodiments, the VR glasses 10 may send the determined light emitting parameter to the light source driver 14 through an I2C bus or the like, to adjust the light emitting parameter of the at least a part of light sources in the light source group 11L/light source group 11R.

S704: Perform eye movement tracking based on the next frame of captured eye image.

After capturing the next frame of eye image, the VR glasses 10 perform eye movement tracking based on the captured eye image. For example, the VR glasses may perform eye movement tracking on the user's eye according to an eye movement tracking algorithm.

According to the method provided in embodiments of this application, the VR glasses 10 may reduce brightness of light sources and reduce a quantity of turned-on light sources in some scenarios, to reduce power consumption of each light source group 11L/light source group 11R and improve a battery life of the VR glasses 10.

It may be understood that, in some embodiments, when the user wears the VR glasses 10, the VR glasses 10 may calibrate human eyes, that is, determine an eye model of the user, for example, a shape, a size, a pupil distance, a refractive power, and astigmatism of the user's eyes. In a process of calibrating the human eyes by the VR glasses 10, the VR glasses 10 usually implements the calibration based on a plurality of eye images according to a calibration algorithm. In some embodiments, in a process of capturing an eye image used for human eye calibration, the VR glasses 10 may alternatively adjust the light emitting parameter of the light source in the light source group 11L/light source group 11R, for example, control the light source to blink, to improve validity of the captured eye image, so as to improve precision of the determined eye model.

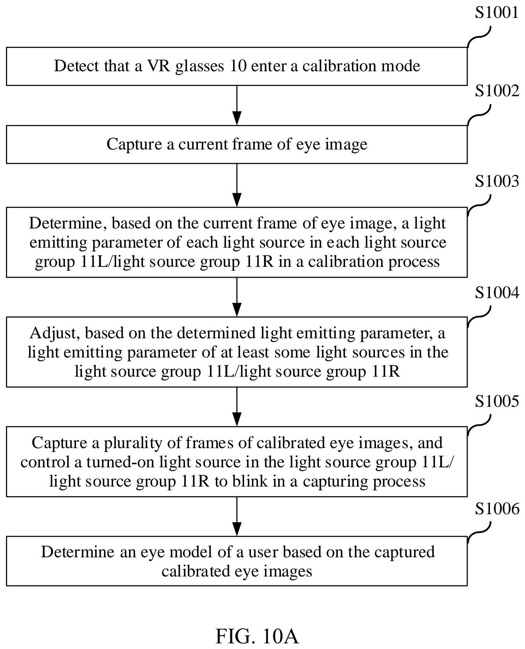

Specifically, FIG. 10A is a schematic flowchart of another eye movement tracking method according to some embodiments of this application. The method may be performed by the VR glasses 10, for example, the processor 110 of the VR glasses 10. As shown in FIG. 10A, the method includes the following steps.

S1001: Detect that the VR glasses 10 enter a calibration mode.

For example, when detecting that a user wears the VR glasses 10 well, the VR glasses 10 may detect that the VR glasses enter the calibration mode, to trigger the eye movement tracking method provided in embodiments of this application.

For another example, when detecting an operation of the user on a button 150, the VR glasses 10 may enter the calibration mode, to trigger the eye movement tracking method provided in embodiments of this application.

For another example, the VR glasses 10 may enter the calibration mode when receiving a calibration instruction sent by another electronic device, for example, a calibration instruction sent by an electronic device connected to the VR glasses 10 by using an application corresponding to the VR glasses 10, and trigger the eye movement tracking method provided in this embodiment of this application.

S1002: Capture a current frame of eye image.

The VR glasses 10 capture the current frame of eye image after entering the calibration mode.

It may be understood that, in some embodiments, if the current frame of eye image is a first frame of eye image captured in a calibration process of the VR glasses 10, when the VR glasses 10 capture the current frame of eye image, the light emitting parameter of the light source in the light source group 11L/light source group 11R may be a preset light emitting parameter of the VR glasses 10. For example, all light sources emit light at same preset brightness.

S1003: Determine a light emitting parameter of each light source in each light source group 11L/light source group 11R in the calibration process based on the current frame of eye image.

After capturing the current frame of eye image, the VR glasses 10 determine the light emitting parameter of each light source in each light source group 11L/light source group 11R in the calibration process based on the current frame of eye image. For a specific determining manner, refer to step S702. This is not limited herein.

For example, if there is an invalid projection in the current frame of eye image, the VR glasses 10 may turn off a light source corresponding to the invalid projection in the calibration process.

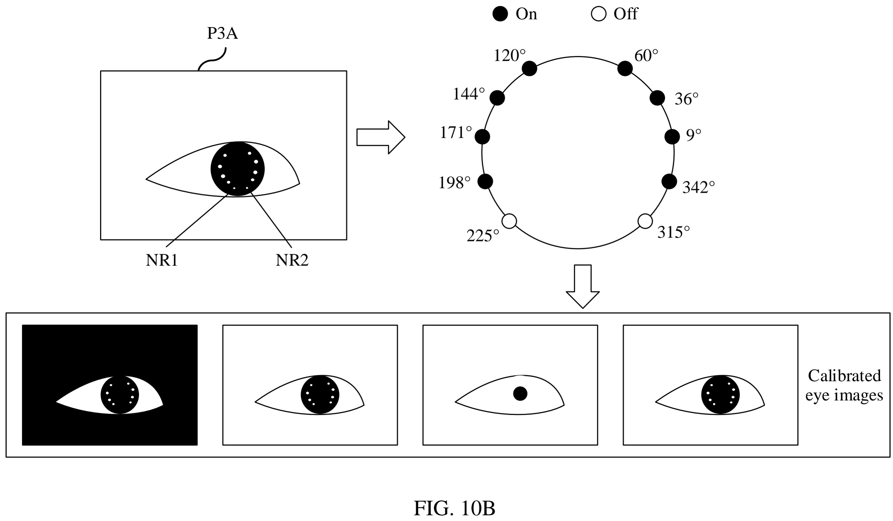

For example, refer to FIG. 10B. For the current frame of eye image P3A shown in FIG. 2B, the VR glasses 10 may determine, based on that there are invalid projections NR1 and NR2 in the current frame of eye image P3A and that the invalid projections NR1 and NR2 respectively correspond to the light source at the position of 225° and the light source at the position of 315°, to turn off the light source at the position of 225° and the light source at the position of 315° in the calibration process.

S1004: Adjust a light emitting parameter of at least a part of light sources in the light source group 11L/light source group 11R based on the determined light emitting parameter.

After determining the light emitting parameter of each light source in the light source group 11L/light source group 11R in the calibration process, the VR glasses 10 adjust the light emitting parameter of the light source in the light source group 11L/light source group 11R to the determined light emitting parameter.

For example, refer to FIG. 10B. The VR glasses 10 may turn off the light source at the position of 225° and the light source at the position of 315°.

S1005: Capture a plurality of frames of calibrated eye image, and control, in a capture process, a turned-on light source in the light source group 11L/light source group 11R to blink.

The VR glasses 10 capture the plurality of frames of calibrated eye image, and control, in the capture process, the turned-on light source in the light source group 11L/light source group 11R to blink.

It may be understood that, in some embodiments, the VR glasses 10 may periodically adjust, by using the light source driver 14, a voltage or a current applied to the turned-on light source, to control the turned-on light source in the light source group 11L/light source group 11R to blink. For example, in one blinking period, the voltage or the current applied to the turned-on light source is first gradually increased and then gradually decreased, or first gradually decreased and then gradually increased, to control the turned-on light source in the light source group 11L/light source group 11R to blink.

It may be understood that, in some embodiments, a blinking frequency of the turned-on light source in the light source group 11L/light source group 11R may be lower than a frequency of capturing the calibrated eye image. In this way, the plurality of frames of captured calibrated eye image may include eye images of the user at different brightness of the light source, to improve validity of the captured eye images, and help improve precision of human eye calibration. For example, if sampling frequencies of the camera 13L and the camera 13R are a, a blinking frequency of the turned-on light source in the light source group 11L/light source group 11R is b (b < a).

For example, refer to FIG. 10B. By controlling light sources other than the light source at the position of 225° and the light source at the position of 315° to blink, the VR glasses 10 may capture a plurality of frames of calibrated eye image with different brightness and contrast. In this way, a case in which calibration precision is affected because the brightness or contrast corresponding to the captured calibrated eye image does not meet a requirement of the calibration algorithm can be avoided.

S1006: Determine an eye model of the user based on the captured calibrated eye image.

The VR glasses 10 determine the eye model of the user based on the captured calibrated eye image.

According to the method provided in embodiments of this application, the VR glasses 10 may reduce brightness of light sources and reduce a quantity of turned-on light sources in some scenarios, to reduce power consumption of each light source group 11L/light source group 11R and improve a battery life of the VR glasses 10. In addition, because the turned-on light sources that are in the light source group 11L/light source group 11R and that are used by the VR glasses 10 to capture the plurality of frames of calibrated eye image blink, the VR glasses 10 can capture eye images of the user at different brightness of the light sources. This helps improve accuracy of the determined eye model.

It may be understood that, in some embodiments, the camera 13L and the camera 13R periodically capture eye images. The following uses an example in which the camera 13R periodically captures the eye image of the user's right eye, to describe a control time sequence of the eye movement tracking method provided in this embodiment of this application.

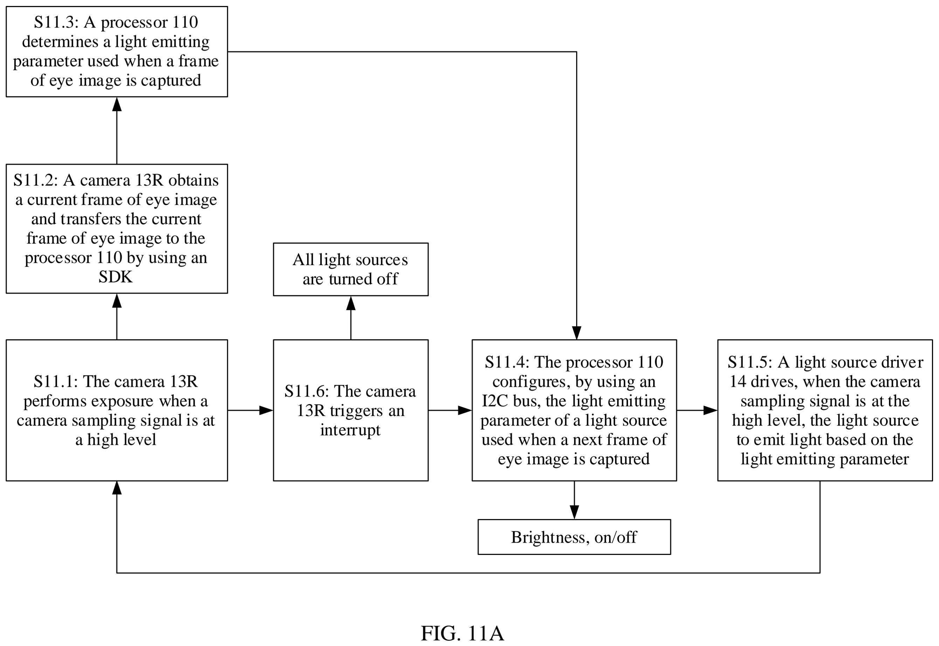

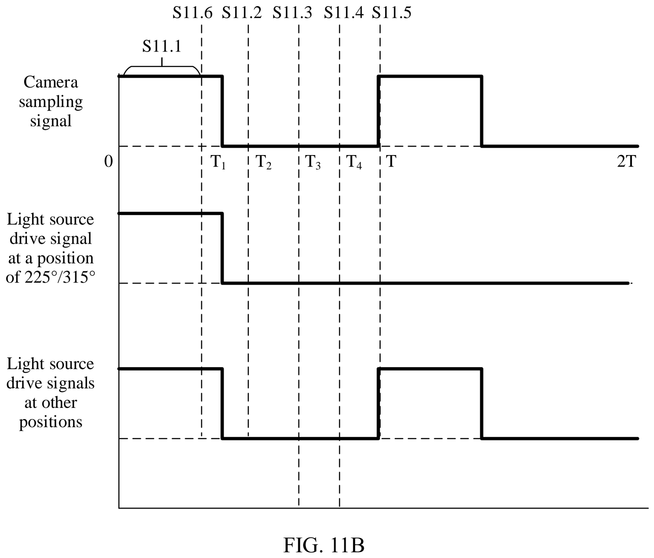

Specifically, FIG. 11A is a diagram of a process of an eye movement tracking method according to some embodiments of this application. FIG. 11B is a diagram of a control time sequence of the eye movement tracking method according to some embodiments of this application.

As shown in FIG. 11A, the process of the eye movement tracking method includes the following steps.

S11.1: The camera 13R performs exposure when a camera sampling signal is at a high level.

For example, the camera 13R performs exposure in a period in which the high level of the camera sampling signal lasts in each period, for example, a period from a moment 0 to a moment T₁ shown in FIG. 11B, to obtain image data corresponding to the eye image.

It may be understood that, in the period in which the camera sampling signal is at the high level, a drive signal corresponding to a light source that needs to be turned on is at the high level. For example, refer to FIG. 11B. For the cases shown in FIG. 2B and FIG. 8A, in the period from the moment 0 to the moment Ti, drive signals of all light sources in the light source group 11R are at high levels, that is, all the light sources are in an on state.

It may be understood that after the exposure is completed, or when the camera sampling signal is switched from the high level to a low level, the camera 13R may switch the drive signals of all the light sources from the high level to the low level, to turn off all the light sources. In this way, in a period in which the camera 13R does not perform exposure, for example, in a period from the moment T₁ to a moment T shown in FIG. 11B, all the light sources are turned off, to save electric energy.

S11.2: The camera 13R obtains a current frame of eye image, and transfers the current frame of eye image to the processor 110 by using an SDK.

After the camera 13R completes the exposure, for example, at a moment T₂ shown in FIG. 11B, the camera 13R may obtain the current frame of eye image, and transfer the current frame of eye image to the processor 110 by using the software development kit (software development kit, SDK).

S11.3: The processor 110 determines a light emitting parameter used when a next frame of eye image is captured.

After receiving the current frame of eye image, for example, at a moment T₃ shown in FIG. 11B, the processor 110 may determine, based on the current frame of eye image, a light emitting parameter, for example, light emitting brightness and on/off, of light sources that are in the light source group 11L and the light source group 11R and that are used when the next frame of eye image is captured. For a specific determining manner, refer to the foregoing step S702 and step S803. Details are not described herein again.

S11.4: The processor 110 configures, by using an I2C bus, the light emitting parameter of the light source used when the next frame of eye image is captured.

After determining the light emitting parameters of the light sources that are in the light source group 11L and the light source group 11R and that are used when the next frame of eye image is captured, for example, at a moment T₄ shown in FIG. 11B, the processor 110 configures the determined light emitting parameter into the light source driver 14 by using the I2C bus. For example, the processor 110 may write the parameters like light emitting brightness and on/off into a register of the light source driver 14 by using the I2C bus.

It may be understood that, in some other embodiments, the processor 110 may alternatively configure, by using another bus or interface, for example, a GPIO of the processor 110, the light emitting parameter of the light source used when the next frame of eye image is captured. This is not limited herein.

S11.5: The light source driver 14 drives, when the camera sampling signal is at the high level, the light source to emit light based on the light emitting parameter.

For example, when detecting that the camera sampling signal corresponding to the next frame of eye image is switched from a low level to a high level, for example, at the moment T shown in FIG. 11B, the processor 110 sets a drive signal of a light source that needs to be turned on and that is determined in step S11.4 to the high level. When detecting the high-level light source drive signal, the light source driver 14 drives, based on the light emitting parameter determined in step S11.4, a corresponding light source to emit light.

For example, in cases corresponding to FIG. 2B and FIG. 8A, the light source driver 14 may detect that drive signals of the light sources at positions of 225° and 315° are switched from the low level to the high level, and turn on light sources other than the light source at the position of 225° and the light source at the position of 315° in the light source group 11R based on drive signals of the light sources at other positions.

S11.6: The camera 13R triggers an interrupt.

When the exposure is completed or when the camera sampling signal is switched from the high level to the low level, the camera 13R may trigger an interrupt to switch drive signals of all light sources from the high level to the low level. In this way, after receiving the interrupt, the light source driver 14 may turn off all light sources in response to the interrupt, to save electric energy.

For a process of adjusting, based on the current frame of the eye image that is of the user's left eye and that is captured by the camera 13L, the light emitting parameter of the light source that is in the light source group 11L and that is used when the camera 13L captures the next frame of eye image, refer to the foregoing steps S11.1 to S11.6. Details are not described herein again.

According to the method provided in this application, in some scenarios, brightness of the light source may be reduced and a quantity of turned-on light sources may be reduced, to reduce power consumption of each light source group 11L/light source group 11R, and improve a battery life of the VR glasses 10. In addition, because the turned-on light sources that are in the light source group 11L/light source group 11R and that are used by the VR glasses 10 to capture the plurality of frames of calibrated eye image blink, the VR glasses 10 can capture eye images of the user at different brightness of the light sources. This helps improve accuracy of the human eye model calibration.

Embodiments of a mechanism disclosed in this application may be implemented in hardware, software, firmware, or a combination of these implementation methods. Embodiments of this application may be implemented as a computer program product or program code executed on a programmable system or device. The programmable system includes at least one processor, a storage system (including a volatile and non-volatile memory and/or storage element), at least one input device, and at least one output device. When the computer program product or program code is executed on the programmable system or device, the programmable system or device may implement the eye movement tracking method provided in embodiments of this application.

The program code can be applied to input instructions to perform the functions described in this application and generate output information. The output information can be applied to one or more output devices in a known manner. For a purpose of this application, a processing system includes any system having a processor like a digital signal processor (DSP), a microcontroller, an application-specific integrated circuit, or a microprocessor.

The program code may be implemented in a high-level programming language or an object-oriented programming language to communicate with the processing system. The program code may alternatively be implemented in an assembly language or a machine language when required. The mechanisms described in this application are actually not limited to the scope of any particular programming language. In any case, the language may be a compiled language or an interpretive language.

In some cases, the disclosed embodiments may be implemented in hardware, firmware, software, or any combination thereof. The disclosed embodiments may alternatively be implemented as instructions that are carried or stored on one or more transitory or non-transitory machine-readable (for example, computer-readable) storage media and that may be read and executed by one or more processors. For example, the instructions may be distributed via a network or through another computer-readable medium. Therefore, a machine-readable medium may include any mechanism for storing or transmitting information in a form readable by a machine (for example, a computer), including, but not limited to, a floppy disk, a compact disc, an optical disc, a magneto-optical disk, a read-only memory (read-only memory, ROM), a random access memory (random access memory, RAM), an erasable programmable read-only memory (erasable programmable read-only memory, EPROM), an electrically erasable programmable read-only memory (electrically erasable programmable read-only memory, EEPROM), a magnetic or optical card, a flash memory, or a tangible machine-readable memory configured to transmit information (for example, a carrier signal, an infrared signal, and a digital signal) through the internet with transmitting signals in electrical, optical, acoustic or other forms. Therefore, the machine-readable medium includes any type of machine-readable medium appropriate for storing or transmitting electronic instructions or information in a machine (for example, a computer)-readable form.

In the accompanying drawings, some structural or method features may be shown in a specific arrangement and/or sequence. However, it should be understood that such a specific arrangement and/or sequence may not be required. In some embodiments, these features may be arranged in a manner and/or a sequence different from those/that shown in the descriptive accompanying drawings. In addition, inclusion of the structural or method features in a specific figure does not imply that such features are required in all embodiments, and in some embodiments, these features may not be included or may be combined with another feature.

It should be noted that all units/modules mentioned in device embodiments of this application are logical units/modules. Physically, one logical unit/module may be one physical unit/module, may be a part of one physical unit/module, or may be implemented by using a combination of a plurality of physical units/modules. Physical implementations of these logical units/modules are not the most important, and a combination of functions implemented by these logical units/modules is a key to resolve the technical problem provided in this application. In addition, to highlight an innovative part of this application, a unit/module that is not closely related to resolving the technical problem provided in this application is not introduced in the foregoing device embodiments of this application. This does not mean that there are no other units/modules in the foregoing device embodiments.

It should be noted that in the examples and specification of this patent, relational terms such as first and second are merely used to distinguish one entity or operation from another, and do not necessarily require or imply any such actual relationship or order between these entities or operations. Furthermore, the term "include" or any other variant thereof is intended to cover a nonexclusive inclusion, so that a process, a method, an article, or a device that includes a list of elements not only includes those elements but also includes other elements that are not expressly listed, or further includes elements inherent to such a process, method, article, or device. Without further limitations, an element limited by "include a/an" does not exclude another same element existing in the process, method, article, or device that includes the element.

## Claims

1. An eye movement tracking method, applied to an electronic device, wherein the electronic device comprises a plurality of light sources, and the method comprises:
capturing a first eye image of a user, wherein the first eye image comprises a projection of at least one of the plurality of light sources onto the user's eye;
adjusting a light emitting parameter of at least a part of the plurality of light sources based on the first eye image; and
capturing a second eye image, and performing eye movement tracking based on the second eye image.

2. The method according to claim 1, wherein the light emitting parameter comprises at least one of the following parameters: light emitting brightness and on/off.

3. The method according to claim 1, wherein the adjusting the light emitting parameter of the at least a part of the plurality of light sources based on the first eye image comprises:
adjusting the light emitting parameter of the at least a part of the plurality of light sources in at least one of the following manners:
adjusting light emitting brightness of the at least a part of the plurality of light sources based on an image parameter of the first eye image, or turn-on/turn-off of the at least a part of light sources, wherein the image parameter comprises contrast;
controlling turn-on or turn-off of the at least a part of the plurality of light sources based on a projection parameter of each projection, wherein the projection parameter comprises at least one of a distribution, an area, brightness, or a shape; and
controlling turn-on or turn-off of the at least a part of the plurality of light sources based on a movement status of the user's eye, wherein the movement status is obtained based on the first eye image.

4. The method according to claim 3, wherein the adjusting the light emitting brightness of the at least a part of the plurality of light sources based on the image parameter of the first eye image, or turn-on/ turn-off of the at least a part of light sources comprises:
when the contrast of the first eye image is greater than first contrast, reducing light emitting brightness of the at least one light source, or turning off a part of light sources in the at least one light source; or
when the contrast of the first eye image is less than second contrast, increasing light emitting brightness of the at least one light source, or increasing a quantity of turned-on light sources.

5. The method according to claim 3, wherein the controlling turn-on or turn-off of the at least a part of the plurality of light sources based on the projection parameter of each projection comprises:
determining an invalid projection in the first eye image, and turning off a light source corresponding to the invalid projection.

6. The method according to claim 5, the determining the invalid projection in the first eye image comprises:
determining a first projection as the invalid projection when the first projection meets at least one of the following conditions:
in the first eye image, a distance between the first projection and a center of the user's eye is greater than a first distance;
in the first eye image, an area of a connected region formed by the first projection and a second projection corresponding to at least one second light source is greater than a first area, or a distance between the connected region and the center of the user's eye is greater than a second distance;
an area of the first projection is less than a second area;
brightness of the first projection is less than first brightness; and
a similarity between a shape of the first projection and a first shape is less than a first similarity.

7. The method according to claim 3, wherein the adjusting the light emitting parameter of the at least a part of light sources in the plurality of light sources based on the movement status of the user's eye comprises:
adjusting a quantity of turned-on light sources in the plurality of light sources to a first quantity when an eye movement manner of the user is fixation; or
adjusting a quantity of turned-on light sources in the plurality of light sources to a second quantity when an eye movement manner of the user is a saccade or a smooth pursuit, wherein the first quantity is less than the second quantity.

8. The method according to claim 3, wherein the method further comprises:
adjusting a quantity of turned-on light sources in the plurality of light sources to a third quantity when a distance between a first position of a pupil center of the user in the first eye image and a second position of the pupil center of the user in a third eye image is less than a third distance; or
adjusting a quantity of turned-on light sources in the plurality of light sources to a fourth quantity when a distance between a first position and a second position is greater than or equal to a third distance, wherein
the third quantity is less than the fourth quantity, and the third eye image is an eye image captured before the first eye image.

9. The method according to claim 1, wherein the second eye image comprises a plurality of frames of eye image, and the method further comprises:
controlling the plurality of light sources to blink in a process of capturing the plurality of frames of eye image, and determining a model of the user's eye based on the second eye image.

10. The method according to claim 9, wherein a frequency at which the plurality of light sources blink is less than a capture frequency of the plurality of frames of eye image.

11. A readable storage medium, wherein the readable storage medium comprises instructions, and when the instructions are executed by an electronic device, the electronic device is enabled to implement the eye movement tracking method according to any one of claims 1 to 10.

12. An electronic device, wherein the electronic device comprises:
a plurality of light sources;
a memory, configured to store instructions; and
a processor, configured to execute the instructions, so that the electronic device controls the plurality of light sources to implement the eye movement tracking method according to any one of claims 1 to 10.

13. A program product, wherein when the program product runs on an electronic device, the electronic device is enabled to implement the eye movement tracking method according to any one of claims 1 to 10.
